# EUROPÄISCHE PATENTSCHRIFT

(11) **EP 0 695 290 B1**
(45) Veröffentlichungstag und Bekanntmachung des Hinweises auf die Patenterteilung: **05.11.1997**
(21) Anmeldenummer: 94915088.2
(22) Anmeldetag: 20.04.1994
(51) Int. Cl.: C07C 311/48, C07D 285/00, C07B 39/00

(54) **N-FLUORSULFONIMIDE, VERFAHREN ZU IHRER HERSTELLUNG SOWIE DEREN VERWENDUNG ALS FLUORIERUNGSMITTEL**
N-FLUOROSULPHONIMIDES, METHOD OF PREPARING THEM AND THEIR USE AS FLUORINATION AGENTS
N-FLUOROSULFONIMIDES, LEUR PROCEDE DE FABRICATION ET LEUR UTILISATION COMME AGENTS DE FLUORATION

(30) Priorität: 20.04.1993 DE 4313664
(43) Veröffentlichungstag der Anmeldung: 07.02.1996
(73) Patentinhaber: SCHERING AKTIENGESELLSCHAFT, 13353 Berlin (DE)
(72) Erfinder: BOHLMANN, Rolf, D-14055 Berlin (DE)
(86) Internationale Anmeldenummer: EP9401251
(87) Internationale Veröffentlichungsnummer: WO9424098

(56) Entgegenhaltungen:
- EP-A- 0 252 431
- US-A- 4 828 764
- US-A- 5 254 732
- SYNLETT Nr. 3 , März 1991 , STUTTGART DE Seiten 187 - 189 E. DIFFERDING 'N-Fluorobenzenesulfonimide: a practical reagent for electrophilic fluorinations' in der Anmeldung erwähnt
- TETRAHEDRON LETTERS Bd. 32, Nr. 13 , 25. März 1991 , OXFORD GB Seiten 1631 - 1634 F.A. DAVIS 'N-Fluoro-o-benzenedisulfonimide: a useful new fluorinating reagent' in der Anmeldung erwähnt
- JOURNAL OF THE AMERICAN CHEMICAL SOCIETY. Bd. 106, Nr. 2 , 25. Januar 1984 , GASTON, PA US Seiten 452 - 454 W.E. BARNETTE 'N-Fluoro-N-alkylsulfonamides: useful reagents for the fluorination of carbanions'
- SYNLETT, Nr. 3, März 1991, Stuttgart, DE, Seiten 187-189, E. DIFFERDING: "N-Fluorobenzenesulfonimide: a practical reagent for electrophilic fluorinations"
- TETRAHEDRON LETTERS, Bd. 32, Nr. 13, 25. März 1991, Oxford, GB, Seiten 1631-1634, F.A. DAVIS: "N-Fluoro-o-benzenedisulfonimide: a useful new fluorinating reagent"

## Beschreibung

Die vorliegende Erfindung betrifft N-Fluorsulfonimide, die als Fluorierungsreagenzien geeignet sind.

Bei der Suche nach neuen pharmazeutischen oder agrochemischen Produkten haben fluorierte Substanzen große Bedeutung. Zur Herstellung dieser Verbindungen spielen elektrophile Fluorierungsreagenzien eine besondere Rolle, da sie den Austausch von aktivierten Wasserstoffen durch Fluor in einem Schritt erlauben. Bekannte Reagenzien dieses Typs sind unter anderen das zu explosiven Reaktionen neigende Gas Perchlorylfluorid, das kaum lagerfähige Cäsiumperoxofluorsulfat, das instabile Acetylhypofluorit, das sehr reaktive Perfluormethansulfonimid, das N-Fluorphenylsulfonimid (E. Differding und H. Ofner, "Synlett", S. 187, 1991) und das N-Fluor-*ortho*-benzodisulfonimid (F. A. Davis und W. Han, "Tetrahedron Letters" 32, S. 1631, 1991).

Obwohl mit dem N-Fluorphenylsulfonimid ein leicht handhabbares, gut zugängliches, reaktives Agens für die elektrophile Fluorierung zur Verfügung steht, ist sein Gehalt von 60,2 g aktivem Fluor/kg Fluorierungsagens (Formelgewicht N-Fluorphenylsulfonimid 315,3 g/Mol) relativ niedrig.

Aufgabe der vorliegenden Erfindung ist die Bereitstellung neuer Verbindungen zur effektiveren Verwendung als Fluorierungsmittel für die elektrophile Fluorierung aktivierter C-H-Bindungen.

Es wurde nunmehr gefunden, daß sich die neuen N-Fluorsulfonimide der allgemeinen Formel I

(R¹-SO₂)₂NF (I),

worin
- R¹: jeweils eine Methylgruppe oder gemeinsam eine Gruppe -(CH₂)ₙ- mit n = 1, 2 oder 3
bedeutet,
einfach herstellen und sich in hervorragender Weise zur elektrophilen Fluorierung aktivierter C-H-Bindungen einsetzen lassen.

Bevorzugt im Rahmen der vorliegenden Erfindung sind das N-Fluormethansulfonimid und N-Fluor[1,3,2]dithiazinan-1,1,3,3-tetraoxid.

Die erfindungsgemäßen Fluorsulfonimide der allgemeinen Formel I weisen aufgrund ihres, verglichen mit N-Fluorphenylsulfonimid, deutlich niedrigeren Formelgewichts, einen höheren aktiven Fluorgehalt auf; beispielsweise hat das erfindungsgemäße N-Fluormethansulfonimid bei einem Formelgewicht von 191,2 g/Mol einen aktiven Fluorgehalt von 99,3 g Fluor/kg.

Die Verbindungen der allgemeinen Formel I sind in vielen organischen Solventien leicht löslich. Das nach der Fluorierung aus der aktiven Verbindung zurückbleibende Sulfonimid besitzt im Vergleich zum Phenylsulfonimid wesentlich bessere Wasserlöslichkeit. Dadurch läßt sich das jeweils gebildete Sulfonimid ganz einfach durch Auswaschen der organischen Phase, beispielsweise Diethylether, in der sich das fluorierte Produkt befindet, abtrennen. Es werden so aufwendige chromatographische Reinigungsschritte und die Verwendung großer Lösungsmittelmengen vermieden.

Es ist als überraschend zu bewerten, daß die erfindungsgemäßen Verbindungen der allgemeinen Formel I wie das bekannte N-Fluorphenylsulfonimid stabil sind. Bei den Verbindungen der allgemeinen Formel I wäre eigentlich zu erwarten gewesen, daß das sehr reaktive Fluor intra- und/oder intermolekular auf den Rest R¹ übertragen würde, daß sich die Verbindungen der allgemeinen Formel I sozusagen selbst fluorieren würden.

Die Herstellung der N-Fluorsulfonimide der allgemeinen Formel I erfolgt erfindungsgemäß durch Umsetzung der leicht zugänglichen Sulfonimide der allgemeinen Formel II

(R¹SO₂)₂NH (II),

worin
- R¹: jeweils eine Methylgruppe oder gemeinsam eine Gruppe -(CH₂)ₙ- mit n = 1, 2 oder 3
bedeutet,
mit elementarem Fluor in einem geeingneten organischen Lösungsmittel.

Als Lösungsmittel dient vorzugsweise Acetonitril. Die Reaktionstemperatur soll zwischen 0 °C und -50 °C liegen; sie beträgt vorzugsweise -40 °C. Die Fluorierung wird vorzugsweise in Gegenwart eines Alkalifluorids, wie beispielsweise Natriumfluorid, durchgeführt, und bei Bedarf kann das Produkt chromatographisch (SiO₂/Eluens z.B. CH₂Cl₂) gereinigt werden.

Vorteilhafterweise können die erfindungsgemäßen N-Fluorsulfonimide zur Fluorierung aktivierter C-H-Bindungen in Aromaten, Enolethern, Enolaten oder Arylaten verwendet werden. Es wird dabei erfindungsgemäß normalerweise so vorgegangen, daß die entsprechende C-H-Bindung zunächst durch Umsetzung des Substrats mit einer starken Base, wie etwa Natriumhydrid, Lithiumdiisopropylamid oder einer Alkyllithiumverbindung wie beispielsweise tert.-Butyllithium, deprotoniert und die so aktivierte C-H-Bindung dann durch Reagierenlassen mit einem N-Fluorsulfonimid der allgemeinen Formel I fluoriert wirt. In Abhängigkeit von der Basenstärke und der Reaktivität der C-H-Bindung wird die Deprotonierung bei einer Temperatur zwischen -20 °C bis +40 °C und die eigentliche Fluorierung bei einer Temperatur zwischen -100 °C bis +20 °C vorgenommen.

Die fluorierten Verbindungen fallen nach Aufarbeitung in sehr hohen Ausbeuten von bis zu 98 % an.

Anhand der nachfolgenden Beispiele wird die vorliegende Erfindung näher erläutert. Die Anwendungsbeispiele sollen die universelle Verwendbarkeit der Verbindungen der allgemeinen Formel I zur elektrophilen Fluorierung C-H-aktivierter Verbindungen demonstrieren.

### Beispiel 1

### N-Fluormethansulfonimid

In eine Lösung von 15 g Dimethylsulfonimid (Helferich und Flechsig, " Berichte" 75, S. 532, 1942) in 200 ml Acetonitril werden bei -40 °C in Gegenwart von 14,5 g Natriumfluoridpulver innerhalb von 2,5 Stunden 60 Liter einer Mischung von 10 Volumenteilen Fluor und 90 Volumenteilen Stickstoff eingeleitet. Anschließend wird mit reinem Stickstoff gespült, im Vakuum eingeengt, mit 200 ml Essigester aufgenommen, über Celite filtriert, im Vakuum eingeengt und aus Essigester/Hexan umkristallisiert. Man erhält 14,9 g N-Fluormethansulfonimid als blaßgelbe Kristalle vom Schmelzpunkt 45 - 48 °C. Bei Bedarf kann über Kieselgel mit Dichlormethan chromatographisch gereinigt werden.

### Beispiel 2

### N-Fluor[1,3,2]dithiazinan-1,1,3,3-tetraoxid

In eine Lösung von 370 mg [1,3,2]Dithiazinan-1,1,3,3-tetraoxid (Geisler und Kuschmiers, "Chem. Ber." 91, S. 1881, 1958) in 23 ml Acetonitril werden bei -40 °C in Gegenwart von 82 mg Natriumfluoridpulver innerhalb von 9 Minuten 0,5 1 einer Mischung von 10 Volumenteilen Fluor und 90 Volumenteilen Stickstoff eingeleitet. Anschließend wird mit reinem Stickstoff gespült, im Vakuum eingeengt, mit Dichlormethan aufgenommen, über 60 g Kieselgel chromatographiert, im Vakuum eingeengt und aus Aceton/Hexan umkristallisiert. Man erhält 331 mg N-Fluormethansulfonimid als Kristalle vom Schmelzpunkt 171 - 172 °C.

### Anwendungsbeispiele

### 2-Fluor-2-phenyl-malonsäurediethylester

Eine Lösung von 1,18 g 2-Phenyl-malonsäurediethylester in 10 ml Dimethylformamid wird mit 200 mg Natriumhydrid (60 % in Öl) 1 Stunde bei 20 °C gerührt, bei 0 °C langsam mit 956 mg N-Fluormethansulfonimid versetzt und 30 Minuten gerührt. Dann wird mit Wasser verdünnt, mit Diethylether extrahiert, mit Wasser gewaschen, über Natriumsulfat getrocknet und im Vakuum eingeengt. Man erhält 1,353 g rohen 2-Fluor-2-phenyl-malonsäurediethylester.

### 2-Fluor-1-phenyl-propan-1-on

Eine Lösung von 268 mg 1-Phenyl-propan-1-on in 1,5 ml Tetrahydrofuran wird bei 0 °C zu 1,3 ml einer 1,6 molaren Lösung von Lithiumdiisopropylamid in Tetrahydrofuran gegeben, 15 Minuten gerührt, auf -78 °C abgekühlt, mit 382 mg N-Fluormethansulfonimid in 4 ml Tetrahydrofuran versetzt und langsam auf Raumtemperatur erwärmt. Dann wird mit Wasser verdünnt, mit Diethylether extrahiert, mit Wasser gewaschen, über Natriumsulfat getrocknet und im Vakuum eingeengt. Man erhält 358 mg rohes 2-Fluor-1-phenyl-propan-1-on.

### 9-Fluoranthracen

Eine Lösung von 514 mg 9-Bromanthracen in 5 ml Tetrahydrofuran/Diethylether (1:1) wird bei -78 °C mit 1,5 ml einer 1,4 molaren tert. Butyllithium-in-Pentan-Lösung versetzt, 30 Minuten bei -78 °C gerührt, mit 382 mg N-Fluormethansulfonimid versetzt, 1 Stunde bei -78 °C gerührt und langsam auf Raumtemperatur erwärmt. Anschließend wird mit Wasser verdünnt, mit Essigester extrahiert, mit Wasser gewaschen, über Natriumsulfat getrocknet und im Vakuum eingeengt. Man erhält 606 mg rohes 9-Fluoranthracen.

## Patentansprüche

1. N-Fluorsulfonimide der allgemeinen Formel I
(R¹-SO₂)₂NF (I),
worin
R¹ jeweils eine Methylgruppe oder gemeinsam eine Gruppe -(CH₂)ₙ- mit n = 1, 2 oder 3
bedeutet.

2. N-Fluormethansulfonimid, (CH₃SO₂)₂NF.

3. N-Fluor[1,3,2]dithiazinan-1,1,3,3-tetraoxid,

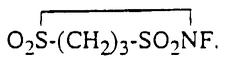

4. Verfahren zur Herstellung der N-Fluorsulfonimide der allgemeinen Formel I
(R¹-SO₂)₂NF (I),
worin
R¹ jeweils eine Methylgruppe oder gemeinsam eine Gruppe -(CH₂)ₙ mit n = 1, 2 oder 3
bedeutet, dadurch gekennzeichnet, daß ein Sulfonimid der allgemeinen Formel II
(R¹SO₂)₂NH (II),
worin
R¹ jeweils eine Methylgruppe oder gemeinsam eine Gruppe -(CH₂)ₙ- mit n = 1, 2 oder 3
bedeutet,
mit elementarem Fluor fluoriert wird.

5. Verfahren nach Anspruch 4, dadurch gekennzeichnet, daß in Acetonitril als Lösungsmittel fluoriert wird.

6. Verfahren nach Anspruch 4 oder 5, dadurch gekennzeichnet, daß bei einer Temperatur zwischen 0 °C und -50 °C, vorzugsweise bei -40 °C, fluoriert wird.

7. Verwendung der N-Fluorsulfonimide der allgemeinen Formel I gemäß Anspruch 1 zur elektrophilen Fluorierung aktivierter C-H-Bindungen organischer Moleküle.

## Claims

1. N-Fluorosulphonimides of the general formula I
(R¹-SO₂)₂NF (I)
wherein
each R¹ represents a methyl group or both together represent a group -(CH₂)ₙ- wherein n = 1, 2 or 3.

2. N-Fluoromethanesulphonimide, (CH₃SO₂)₂NF.

3. N-Fluoro[1,3,2]dithiazinane-1,1,3,3-tetraoxide,

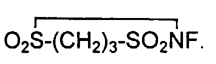

4. Process for the preparation of N-fluorosulphonimides of the general formula I
(R¹-SO₂)₂NF (I)
wherein
each R¹ represents a methyl group or both together represent a group -(CH₂)ₙ- wherein n = 1, 2 or 3,
characterised in that a sulphonimide of the general formula II
(R¹SO₂)₂NH (II)
wherein
each R¹ represents a methyl group or both together represent a group -(CH₂)ₙ- wherein n = 1, 2 or 3,
is fluorinated with elemental fluorine.

5. Process according to claim 4, characterised in that the fluorination is carried out in acetonitrile as solvent.

6. Process according to claim 4 or 5, characterised in that the fluorination is carried out at a temperature of from 0°C to -50°C, preferably at -40°C.

7. Use of the N-fluorosulphonimides of the general formula I according to claim 1 for the electrophilic fluorination of activated C-H bonds of organic molecules.

## Revendications

1. N-fluorosulfonimides de formule générale I
(R¹-SO₂)₂NF (I)
où
R¹ chacun représente un groupe méthyle ou ensemble un groupe -(CH₂)ₙ- avec n = 1, 2 ou 3.

2. N-fluorométhanesulfonimide, (CH₃-SO₂)₂NF .

3. 1,1,3,3-tétroxyde de N-fluoro[1,3,2]dithiazinane,

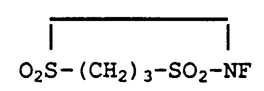

4. Procédé de préparation de N-fluorosulfonimides de formule générale I
(R¹-SO₂)₂NF (I)
où
R¹ chacun représente un groupe méthyle ou ensemble un groupe -(CH₂)ₙ- avec n = 1, 2 ou 3,
caractérisé en que l'on effectue avec du fluor élémentaire la fluoration d'un sulfonimide de formule générale II
(R¹-SO₂)₂NH (II)
R¹ chacun représente un groupe méthyle ou ensemble un groupe -(CH₂)ₙ- avec n = 1, 2 ou 3.

5. Procédé selon la revendication 4, caractérisé en ce que l'on met en oeuvre la fluoration en milieu d'acétonitrile en tant que solvant.

6. Procédé selon la revendication 4 ou 5, caractérisé en ce que l'on réalise la fluoration à une température comprise entre 0°C et -50°C, de préférence à -40°C.

7. Utilisation des N-fluorosulfonimides de formule générale I selon la revendication 1, pour la fluoration électrophile des liaisons C-H activées des molécules organiques.
